# FASCICULE DE BREVET EUROPEEN

(11) **EP 1 792 610 B1**
(45) Date de publication et mention de la délivrance du brevet: **15.10.2008**
(21) Numéro de dépôt: 07006150.2
(22) Date de dépôt: 23.07.2002
(51) Int. Cl.: A61K 9/00, A61K 9/20

(54) **Systèmes thérapeutiques bioadhésifs à libération prolongée**
Bioadhäsive therapeutische Systeme mit verzögerter Freisetzung
Sustained release bioadhesive therapeutic systems

(30) Priorité: 23.07.2001 FR 0109811
(43) Date de publication de la demande: 06.06.2007
(62) Demande divisionnaire de: 02774826.8
(73) Titulaire: BioAlliance Pharma, 75015 Paris (FR)
(72) Inventeur: Aiache, Jean-Marc, 63000 Clermont-Ferrand (FR); Constantini, Dominique, 75014 Paris (FR); Chaumont, Christine, 31400 Toulouse (FR)
(74) Mandataire: Desaix, Anne

(56) Documents cités:
- WO-A-92/02209
- US-A- 4 873 080

## Description

La présente invention concerne le domaine médical, et en particulier le domaine des infections mucosales. La présente invention concerne plus particulièrement de nouveaux systèmes thérapeutiques bioadhésifs à libération prolongée utiles pour le traitement des infections mucosales locales, de type mucite et candidose.

La candidose est une pathologie résultant d'une prolifération locale d'espèces fongiques (Candida). Cette prolifération peut avoir plusieurs origines, et notamment un déséquilibre physico-chimique local (modification du pH, etc), lié par exemple à la prise d'antibiotiques, de stéroïdes ou d'autres traitements physiques (irradiation, chimiothérapie, immunosuppresseurs, etc). L'augmentation des situations d'immunodépression (en relation avec les immunosuppressions consécutives à des chimiothérapies dans le traitement du cancer ou à l'épidémie de SIDA) a été à l'origine d'une recrudescence de candidoses orales et de pathologies parodontales sévères (Hermant et al., 1997, Med. Mal. Infect. 27:715-718). Les candidoses buccales sont couramment détectées même chez les sujets séropositifs pour le HIV apparemment en bonne santé. Elles sont souvent la première manifestation d'une infection HIV. Plus de 90 % des patients avec un SIDA développent une candidose orale (Vasquez, 1999, Pharmacotherapy, 19 (1) : 76-87). La prévalence approche les 20 % dans certaines populations, elle augmente avec la diminution du nombre de CD4 (Greenspan & Greenspan, 1996, Lancet 348:729-733). En outre, la candidose est une caractéristique commune de l'infection par le virus d'immunodéficience VIH et du cancer. Les espèces fongiques responsables de ces candidoses sont notamment les Candida tels que C. albicans C. glabrata, C. tropicalis ou encore C. krusei.

Les signes associés de la candidose sont en particulier une sécheresse de la bouche, des douleurs à l'ingestion, une perte de goût, des brûlures, etc. Une altération de l'état de la cavité buccale a des implications sérieuses sur l'état général du patient. Les infections buccales mal traitées peuvent être à l'origine d'odynophagie et de dysphagie, pouvant interférer avec la parole, la mastication et la déglutition. De plus, les douleurs provoquées par ces infections conduisent les patients à réduire leur alimentation ; il en résulte une perte de poids, une déshydratation et une malnutrition. La prévention et le traitement des candidoses orales est donc une préoccupation essentielle pour le maintien de la qualité de vie et la prévention des complications plus sévères chez ces patients (Weinert et al., 1996, Amn. Intern. Med. 125: 485-496).

La candidose est également une complication fréquente des traitements anticancéreux. En particulier, les traitements de chimiothérapie, les greffes de moelle ou l'irradiation locale sont autant de facteurs favorisant le développement des infections locales de type candidose. Les effets secondaires oraux de la chimiothérapie sont source de morbidité importante en cancérologie. Sur 27 essais cliniques: 14 avec des mucites (945 cas randomisés) et 15 avec des candidoses orales (1164 cas randomisés), l'incidence des mucites est de 50 à 80 % et celle des candidoses est de 30 à 70 % et varie en fonction de la localisation du cancer. Au vu de cette analyse, les traitements partiellement absorbés semblent plus efficaces que ceux qui sont totalement absorbés par le tractus intestinal en ce qui concerne la prophylaxie (Clarkson et al., 2000, Cochrane Database Syst Rev, (2) : CD000978).

De manière plus générale, un autre facteur favorisant le développement de candidoses est l'altération de l'intégrité de la muqueuse, par exemple par desquamation locale ou diffuse. La mucite est une de ces conditions : il s'agit d'un érythème généralement suivi d'une desquamation locale. L'altération de la muqueuse (notamment buccale) ainsi induite est le stade précoce faisant le lit de l'infection notamment fongique dans le SIDA ou le cancer (chimiothérapie, greffe de moelle, irradiation locale lors de tumeurs de la tête et du cou, etc). Les patients souffrent localement d'une hypofonction salivaire, elle même responsable d'une altération d'hygiène dentaire (Greenspan et al., The Lancet 348 (1996) 729).

Actuellement, les infections candidosiques sont traitées en première intention de manière locale, essentiellement par des antifongiques : préparations magistrales, comprimés à sucer, bains de bouche, azolés, polyènes (Greenspan et al., Lancet 348:729-733). Des dérivés azolés sont proposés en deuxième intention, par voie systémique, en cas de candidose oesophagienne (kétoconazole, fluconazole, itraconazole). Ces traitements sont efficaces en prévention mais présentent des risques importants (interactions médicamenteuses, résistance, intolérance).. Ces azolés systémiques sont réservés pour des thérapies courtes dans les candidoses avérées (Kovacs et al, 2000, The New England Journal of Medicine, May 11, 1416-1429).

Un autre inconvénient de ces traitements réside dans les rechutes fréquemment observées. Ainsi, certaines études montrent 60% de récurrence dans les trois mois suivant le traitement (Imam et al., Amer. J. of Medicine 89 (1990) 142).

Si on considère la physiopathologie de la candidose, le *Candida albicans* mais aussi *C*. *krusei, C. tropicalis* et *C*. *glabrata* sont les agents responsables de l'infection locale (bouche, oesophage, peau, ongles, vagin). Celle-ci peut être plus ou moins profonde en fonction des défenses de l'hôte. Il s'agit d'une affection cutanéo-muqueuse pouvant se compliquer et devenir systémique. Traiter efficacement localement ou prévenir les candidoses buccales peut éviter les candidoses systémiques et l'apparition de souches résistantes.

L'adhésion du champignon à la muqueuse est notamment un élément essentiel de sa pathogénicité. Le temps de résidence d'un produit antifongique dans la cavité buccale peut être un élément essentiel à son efficacité immédiate et à long terme.

Les formulations ou compositions décrites ou existantes portent sur des formes à administration systémiques, tant quant à leur forme galénique qu'aux principes actifs utilisés.

La présente invention fournit un système thérapeutique bioadhésif à libération prolongée, essentielle pour privilégier un temps de présence long sur les lieux de l'infection à l'inverse des formes locales habituelles (bains de bouche, gel, pastilles, comprimés à sucer) qui ont un effet transitoire.

A titre d'exemple, un gel buccal pour application locale à base de miconazole est commercialisé par la société Janssen-cilag (92787 lssy-les-Moulineaux, France), sous le nom de Daktarin gel buccal ® pour le traitement des mycoses de la cavité buccale. Le miconazole est un antifongique appartenant à la classe des imidazolés. Son action s'exerce *in situ* après application. Etant très peu résorbé, il est bien toléré. La posologie pour le traitement des mycoses buccales chez l'adulte est de 125 mg de miconazole (deux cuillères-mesures) 4 fois par jour en application pendant 7 à 15 jours. La concentration salivaire du miconazole est un bon reflet de l'efficacité du produit. Or elle varie de 5 à 0,4 µg/ml de 30 minutes à 3 heures suivant l'application du gel. Sa diminution très rapide s'explique par le temps de résidence très court du gel dans la cavité buccale. Par ailleurs, la CMI (concentration minimale inhibitrice) du miconazole vis-à-vis de *Candida albicans* est comprise entre 1 et 10 µg/ml (Monographie du Daktarin, information médicale du Compendium Suisse des Médicaments). Cette concentration n'est obtenue que durant les temps courts qui suivent l'application du gel. Par conséquent, la couverture antimycosique obtenue avec le gel buccal est médiocre.

Des formes bioadhésives et leur procédé de préparation sont décrits dans le brevet EP 0542 824 B1. Elles ont été conçues pour un passage systémique et ne sont pas utilisables pour une action locale. Elles ne permettent pas en effet une dissolution *in vitro* satisfaisante, critère d'appréciation d'une disponibilité locale. Elles sont donc incompatibles avec la mise en oeuvre d'un principe actif, tel un antifongique pour lequel sont recherchés une action locale et/ou un passage systémique limité.

La présente invention vise à surmonter l'ensemble des inconvénients décrits ci-avant en proposant de nouveaux systèmes thérapeutiques bioadhésifs à libération prolongée en assurant une solubilisation des principes actifs pour assurer son utilité locale. En effet, les formes à libération prolongée permettent de réduire le nombre de prises, et d'obtenir des taux de principe actif plus stables dans le temps.

Dans l'ensemble du texte, on utilisera indifféremment les termes "bioadhésifs" ou "mucoadhésifs", les systèmes thérapeutiques selon l'invention étant plus particulièrement adaptés à l'administration mucosale.

De la même façon, le terme "thérapeutique" englobe tant la thérapeutique que la prophylaxie des différentes pathologies évoquées ci-dessous et notamment des mucites et candidoses.

Les systèmes thérapeutiques bioadhésifs peuvent être sous forme de comprimés, de microsphères ou de nanosphères bioadhésives.

La présente invention vise également un procédé de préparation desdits systèmes thérapeutiques bioadhésifs sous forme de comprimés conférant à ces derniers les qualités recherchées pour une utilisation sur tous les types de muqueuses.

Plus précisément, les comprimés bioadhésifs et méthodes de l'invention sont adaptées au traitement des mucites et des candidoses dans le cadre de pathologies de sujets immunodéprimés (sujets âgés, malnutris, antibiothérapie, cancer, SIDA, radiothérapie, chimiothérapie, greffe).

Lesdits comprimés sont également adaptés à l'administration de principes actifs dont l'administration par voie mucosale présente des avantages d'ordres thérapeutique ou de confort, par rapport à des administrations par voie buccale, transdermique ou systémique. Il peut s'agir à titre d'exemple des antiviraux tels l'aciclovir, le valaciclovir, le ganciclovir, la zidovudine ou des analgésiques insolubles tels le fentanyl base.

Ils permettent un usage local privilégié de principe actif insolubles ou peu solubles. Ils permettent également d'associer dans les comprimés plusieurs principes actifs visant à augmenter la compliance et l'acceptabilité du traitement, tels d'autres antifongiques à spectre différent, des analgésiques, des agents de salivation, etc.

Les comprimés de l'invention permettent également une réduction des doses et donc des effets secondaires indésirables qui peuvent accompagner l'usage habituel de tels médicaments. Par ailleurs, l'invention décrit également des compositions particulièrement avantageuses sur le plan clinique car actives sur la base d'une seule (voire deux) prise journalière. Ils visent enfin, par leur nouvelle formulation, à ne pas altérer le goût et l'appétit qui sont des éléments essentiels au maintien du bon état général du patient immunodéprimé.

La présente invention apporte ainsi une solution aux inconvénients de l'art antérieur, permettant une prise en charge plus complète et mieux tolérée des infections mucosales et des symptômes associés (couverture continue et non transitoire) ainsi que de certaines pathologies virales, de la douleur ou autres pathologies.

Un premier aspect de l'invention concerne un système thérapeutique bioadhésif mucosal à libération prolongée contenant au moins un principe actif, présentant un test de dissolution du principe actif supérieur à 70 % à 8 heures, comprenant des quantités de protéines naturelles représentant au moins 50 % en poids de principe actif et au moins 20 % en poids dudit système thérapeutique bioadhésif, entre 10 et 20 % d'un polymère hydrophile, des excipients de compression pour renforcer la dureté du système thérapeutique bioadhésif, et comprenant entre 3,5 et 10 % d'un alkylsulfate d'un métal alcalin et optionnellement entre 0,1 % et 1 % d'un sucre monohydrate.

Une forme particulière du système thérapeutique bioadhésif mucosal selon l'invention est un comprimé mucoadhésif.

Le rôle essentiel de l'ajout de l'alkylsulfate de métal alcalin est d'une part de permettre la solubilisation d'un principe actif peu soluble ou insoluble et d'autre part de faciliter sa disponibilité locale: l'agent alkylsulfate de métal alcalin facilite le passage systémique de principe actif en fonction de sa concentration (Martin-Algarra, 1994, Pharmaceutical Research, (11) 7 : 1042-1047). Dans la formulation selon la présente invention, par formation de micelles, il joue un rôle de solubilisant ne facilitant pas l'absorption. Il facilite également le gonflement et permet une libération constante du principe actif sur 8 heures.

Les systèmes thérapeutiques bioadhésifs selon l'invention sont plus particulièrement adaptés à la prévention ou au traitement des infections fongiques buccales, oesophagiennes ou vaginales.

Encore plus particulièrement, ils sont adaptés à la prévention ou au traitement des infections candidosiques buccales chez les patients immunodéprimés. Les comprimés de l'invention sont utilisables à titre curatif ou préventif. Par ailleurs, les comprimés de l'invention sont plus particulièrement destinés à une administration buccale mais également adaptée à une autre voie en adaptant la forme galénique.

En résumé, les systèmes thérapeutiques bioadhésifs selon l'invention présentent plusieurs propriétés et avantages importants :
- une formulation permettant une prise aisée (compatible avec un traitement préventif ou d'entretien) et une activité optimale des principes actifs ;
- un ou plusieurs autres principes actifs et/ou excipients peuvent être associés au premier principe actif pour assurer une hydratation et une bonne acceptation locale des compositions ;
- une dose de principe actif bien inférieure à celle utilisée dans les autres formes galéniques existantes permettant la diminution, voire la suppression des effets indésirables ;
- des concentrations salivaires de principe actif supérieures à la concentration minimale inhibitrice (CMI) pendant un temps prolongé.

Les systèmes thérapeutiques bioadhésifs selon l'invention contiennent au moins un principe actif.

Un principe actif préféré lorsque les systèmes thérapeutiques bioadhésifs sont destinés à la prévention et au traitement des infections fongiques est un composé de la famille des azolés à large spectre, choisi de préférence parmi le miconazole, le clotrimazole, le kétoconazole, le fluconazole, l'itraconazole, l'isoconazole, l'econazole, le saperconazole, le genaconazole, le terconazole, le butoconazole, le tioconazole, l'oxiconazole, le bifonazole, le fenticonazole, l'omoconazole, le sertaconazole, le voriconazole et le sulconazole. Il s'agit avantageusement de triazoles, tels que le fluconazole, l'itraconazole ou le saperconazole ; ou d'imidazoles, choisis de préférence parmi le miconazole, le clotrimazole et le kétoconazole.

Des composés azolés particulièrement préférés au sens de l'invention sont le miconazole, le kétoconazole et l'itraconazole, et les doses unitaires sont alors comprises entre 10 et 150 mg par comprimé.

Un composé encore préféré est le miconazole dont la dénomination chimique est (RS)-1-[-2-(2,4-dichlorobenzyloxy)-2-(2,4-dichlorophenyl)-ethyl]-1H-imidazole, présent à la dose de 10 à 150 mg par comprimé, de préférence 25 à 75 mg et encore de préférence 50 mg par comprimé.

Les composés azolés sont connus pour agir sur la synthèse d'un constituant de la membrane des champignons : l'ergostérol.

Les systèmes thérapeutiques bioadhésifs selon l'invention et contenant sensiblement 50 mg par comprimé assurent une présence continue et prolongée du principe actif supérieure à la CMI sur le site d'action à raison d'une prise par jour ainsi que les différents avantages décrits ci-avant, et l'absence de passage systémique.

Un autre avantage des systèmes thérapeutiques bioadhésifs comprenant sensiblement 50 mg de miconazole, est l'excellente tolérance du principe actif car la dose utile par jour dans la préparation de 50 mg est dix fois plus faible que la dose usuelle du même produit dans une formulation de référence Daktarin gel buccal (500 mg/j), alors que de façon surprenante les concentrations locales sont considérablement accrues (7 à 10). Un avantage important est la diminution du risque d'acquisition de résistances des champignons. En effet, l'apparition de résistance à un composé se produit souvent si la concentration locale est inférieure à la CMI permettant à la levure de croître à nouveau. La dose -utile dans les comprimés de l'invention permet également de diminuer voir éliminer certains effets indésirables résultat d'un dosage à 500 mg/j, tels des troubles intestinaux (nausées, vomissements, diarrhées), ou des réactions allergiques. Des élévations de transaminases ont rarement été observées.

Les différents avantages des systèmes thérapeutiques bioadhésifs selon l'invention apparaissent clairement dans les exemples décrits ci-après, décrivant essentiellement les formes pharmaceutiques et les résultats cliniques comparatifs.

Le composé azolé, du type miconazole, peut être associé à un autre principe actif choisi par exemple parmi un antifongique à spectre différent, de type polyène, un analgésique, un agent de salivation, un substitut salivaire, un antiseptique, un anti-inflammatoire (corticoïde), la thalidomide, ou un mélange de ceux-ci.

Les polyènes ont un mécanisme d'action différent des azolés. Ils se lient aux groupes stérols, principalement ergostérol, présents dans la membrane des champignons et induisent l'apparition de pores et de canaux. Ces pores et canaux augmentent fortement la perméabilité cellulaire et la fuite de petites molécules, conduisant à la mort de la cellule.

Parmi les polyènes, on utilise préférentiellement dans les compositions de l'invention les polyènes à large spectre, et de préférence les tétraènes. On peut citer plus particulièrement la nystatine, et l'amphotéricine B.

On utilise avantageusement dans les systèmes thérapeutiques bioadhésifs de l'invention la nystatine.

Les polyènes sont utilisés avantageusement à des doses comprises entre 10 et 100 mg, de préférence entre 20 et 90 mg. A titre illustratif, l'amphotéricine est avantageusement utilisée dans des doses comprises entre 20 et 60 mg. La nystatine est quant à elle utilisée à raison de 10 mg à 100 mg, de préférence 20 mg à 40 mg, encore plus préférentiellement aux environs de 25 mg.

Les doses peuvent être préconisées en Unités, sachant que 1 mg correspond à 4400 Unités. Une composition préférée au sens de l'invention comprend de 50 à 400 mg d'un composé imidazolé ou triazolé et de 20 à 90 mg ou 100 000 à 200 000 Unités d'un polyène. Encore plus préférentiellement, le composé azolé est le miconazole et le polyène est la nystatine. Les compositions de l'invention sont généralement destinées à une ou deux prises journalières. De préférence, les administrations sont effectuées à un rythme permettant la prise quotidienne de doses allant de 20 à 200 mg d'azolé et de 50 000 à 500 000 Unités de polyène (10 à 110 mg).

Par ailleurs, pour améliorer l'efficacité du traitement, les systèmes thérapeutiques bioadhésifs de l'invention appliqués à l'administration mucosale peuvent comporter en outre un ou plusieurs autres principes actifs. Plus particulièrement, les systèmes thérapeutiques bioadhésifs de l'invention peuvent comprendre un ou plusieurs principes actifs permettant d'assurer une hydratation et une acceptation locale, gage de l'observance dans ce type de traitement. L'emploi de ce type de principe actif est particulièrement avantageux en application aiguë et en traitement d'entretien.

Les excipients et autres principes actifs associés peuvent à titre d'exemple être choisis avantageusement parmi un ou plusieurs composés anesthésiques, agent de salivation, antiseptique, anti-inflammatoire, thalidomide, antibiotique, substituts salivaires, agent de masquage de goût, seuls ou en combinaisons.

L'utilisation combinée d'un principe actif ayant des propriétés anesthésiques est particulièrement avantageuse dans la mesure où, comme indiqué ci-avant, la candidose est souvent accompagnée de douleurs importantes, notamment lors de l'ingestion. Un anesthésique approprié pour une utilisation dans le cadre de l'invention est par exemple la lidocaïne ou la tétracaïne. L'anesthésique est de préférence utilisé à des doses comprises entre 0,1 et 10% en poids du poids total des constituants actifs des compositions de l'invention, encore plus préférentiellement entre 1 et 7%.

Une composition type au sens de l'invention comprend environ de 1 à 5% d'anesthésique, par exemple de lidocaïne.

L'utilisation combinée d'un agent facilitant la salivation est également avantageuse pour le traitement de la candidose, qui induit fréquemment une sécheresse de la bouche. Un autre objet particulier de l'invention est donc relatif à un système thérapeutique bioadhésif ou une composition comprenant au moins un principe actif et un agent de salivation.

Le principe actif peut être un composé antifongique tel que décrit ci-dessous, un antiviral pour le traitement des infections à VIH (virus de l'immunodéficience humaine), à EBV (Virus Epstein-Barr, mononucléose infectieuse, leucoplasie chevelue), CMV (cytomégalovirus), de l'herpès (HSV), ou du virus de la varicelle et du zona (VZV). Les principes actifs contenus dans les systèmes thérapeutiques bioadhésifs sont alors la zidovudine, l'aciclovir, le valaciclovir ou le ganciclovir. Il peut être également un analgésique peu ou non soluble dont les autres modes d'administration posent précisément des problèmes de solubilité. On peut citer, à titre d'exemple, le fentanyl base peu soluble, et intéressant pour le traitement des douleurs intenses, résistantes et notamment associées au cancer.

Un agent de salivation approprié pour une utilisation dans le cadre de l'invention est par exemple la pilocarpine. La pilocarpine est utilisée actuellement dans le traitement de la xérostomie chez les patients recevant une irradiation dans le cadre de traitements de cancers de la tête et du cou. D'autres agents de salivation sont par exemple le béthanechol.

L'agent de salivation est de préférence utilisé à des doses comprises entre 0,1 et 10% en poids du poids total des constituants actifs des compositions de l'invention, encore plus préférentiellement entre 0,1 et 5%.

Une composition type au sens de l'invention comprend environ de 0,5 à 3% d'agent de salivation, par exemple de pilocarpine.

Une association particulière de l'invention comprend plus préférentiellement au moins un composé antifongique, un anesthésique et un agent de salivation. Plus préférentiellement, une composition avantageuse de l'invention comprend :
- de 25 à 75 mg d'un composé imidazolé ou triazolé,
- de 1 à 7% en poids d'un anesthésique, et,
- de 0,1 à 5% en poids d'un agent de salivation.

Par ailleurs, pour améliorer l'observance du traitement, un agent de masquage de goût quand nécessaire, par exemple avec les antifongiques, peut être utilisé. Néanmoins à la dose de 50 mg de miconazole, le comprimé bioadhésif se caractérise par une absence de goût désagréable. Cet agent est de préférence dépourvu de sucre pour éviter les complications possibles au niveau buccal et notamment dentaire (caries). Dans un autre mode de réalisation, les systèmes thérapeutiques bioadhésifs de l'invention comprennent au moins un composé antifongique et un agent de masquage de goût. Ce dernier comprend par exemple des dérivés de type mentholé. L'agent de masquage de goût est avantageusement utilisé en combinaison avec l'anesthésique et l'agent de salivation tels que décrits ci-dessus.

D'autre part, comme indiqué ci-avant, l'utilisation combinée d'un antiseptique présente en outre des avantages importants, notamment pour des traitements prophylactique ou d'entretien. L'emploi de ce type de composés permet en effet d'assurer une meilleure hygiène orale aux patients atteints d'infections candidosiques. En particulier, l'emploi de ce type d'agent permet de réduire les populations bactériennes présentes dans la cavité orale. Un antiseptique approprié est soit l'alkylsulfate de métal alcalin seul, soit associé à la chlorexidine (0 à 5 %), à un antibiotique (la fusafungine à 500 mg par exemple), soit à un anti-inflammatoire, soit à un corticoïde.

L'emploi combiné de ces principes actifs symptomatiques confère aux compositions de l'invention des propriétés supplémentaires, et notamment :
- une hydratation de la muqueuse et de la bouche
- un goût masqué
- un confort aux sujets (prise en charge des douleurs)
- une meilleure hygiène orale.

La présente invention montre ainsi qu'il est possible de combiner dans des compositions actives différents agents ayant des propriétés complémentaires, assurant aux patients un traitement plus efficace des infections candidosiques. En particulier, les compositions de l'invention permettent un traitement global de ces pathologies, par opposition aux traitements systémiques précoces ou locaux par les azolés, dont l'efficacité est bonne mais qui peuvent entraîner l'apparition de résistances.

Les principes actifs utilisés dans le cadre de l'invention peuvent être conditionnés dans un même système thérapeutique bioadhésif homogène ou hétérogène avec deux phases à deux vitesses de libération, ou séparément, selon que l'administration est effectuée simultanément ou de manière espacée dans le temps. Par ailleurs ils peuvent être formulés de différentes manières, selon la nature des composés, et la posologie. La formulation peut être sous forme de comprimés, homogènes ou bi-couches, ou sous forme de micro ou nanosphères. D'une manière générale, le conditionnement et la formulation sont définis de manière à permettre la compatibilité des produits associés, une fréquence d'administration réduite ou facilitée (une à deux prises par jour), un mode d'administration aisé (forme bioadhésive orale de préférence), un goût masqué si nécessaire, une hydratation locale, une absence de passage systémique et une bonne acceptabilité.

Un autre aspect des systèmes thérapeutiques bioadhésifs de l'invention porte sur les excipients et les produits de charge. L'adhésivité est conférée par des protéines naturelles, qui représentent au moins 50 % en poids de principe actif. Les types de protéines naturelles utilisables sont ceux décrits dans EP 0542 824. Un exemple particulier est un concentré de protéines de lait titrant au minimum 85 % de protéines comme par exemple le Prosobel L85, le LR85F ou de préférence soit le Promilk 852A commercialisé par Armor Protéines, soit parmi la gamme Alaplex (4850, 1180, 1380 ou 1395) de NZMP. La concentration relative des protéines naturelles dans un comprimé bioadhésif selon l'invention est de 15 à 50 %, de préférence 20 à 30 %.

Les systèmes thérapeutiques bioadhésifs selon l'invention comprennent également les excipients habituels des systèmes bioadhésifs comme il apparaît dans les exemples ci-après.

Une caractéristique particulière des composés de l'invention est qu'ils contiennent entre 3,5 et 10 % d'un alkylsulfate de métal alcalin. De façon préférée, celui-ci est choisi dans un groupe comprenant le laurilsulfate de sodium ou le diethyl-sulfosuccinate. De façon encore plus préférée, il s'agit du laurilsulfate de sodium présent à la concentration de 4 à 6 % en poids de l'ensemble du comprimé.

La présence dans les comprimés d'une dose supérieure à 3,5 % d'un alkylsulfate de métal alcalin et plus particulièrement de laurilsulfate de sodium permet, comme il est montré dans les exemples ci-après, d'augmenter la libération du miconazole à partir du comprimé *in vitro,* à savoir plus de 80 % à 8 heures contre une impossibilité de dissoudre le principe actif en l'absence d'alkysulfate de métal alcalin y compris dans les temps courts. Cela permet donc d'augmenter la solubilisation de principes actifs peu solubles ou insolubles. Ceci est essentiel à une bonne disponibilité du principe actif localement. Il permet en outre un gonflement supérieur du comprimé, ce qui présente l'avantage d'une libération constante du principe actif dans le site d'action des agents pathogènes. Il est à noter qu'une telle concentration de laurilsulfate de sodium associée au comprimé adhésif est totalement inhabituelle pour un comprimé dans lequel la concentration de ce produit dépasse rarement 3 %.

Ainsi, dans la présente invention, la fonction solubilisante du laurilsulfate de sodium vis-à-vis d'un principe actif peu soluble a un rôle essentiel pour sa libération *in situ.* Les exemples ci-après indiquent une bonne cohérence entre le test de dissolution *in vitro* et la concentration salivaire cumulée au cours du temps mesuré *in vivo.*

Ainsi, la présence de laurilsulfate de sodium à la concentration de 4 à 6 % est un élément essentiel dans la composition du comprimé et dans les qualités du comprimé citées ci-avant : bonne disponibilité, une seule prise par jour, etc.

Le laurilsulfate de sodium à la concentration minimale de 3,5 % et de préférence entre 3,5 et 10 % a l'avantage également de présenter des propriétés antiseptiques en tant que telles. II assure donc non seulement un rôle d'excipient, mais également de principe actif, notamment pour le traitement des mucites.

Les systèmes thérapeutiques bioadhésifs selon l'invention peuvent comprendre enfin entre 0,1 % et 1 % d'un sucre monohydrate et de préférence du lactose monohydrate ou de saccharose. La présence de lactose n'est a priori pas indispensable en tant que telle dans la mesure où la concentration en protéines de lait est importante. Néanmoins, la présence de lactose monohydrate ou de saccharose résulte d'une modification substantielle d'une étape du procédé de préparation telle que décrite dans le brevet EP 0542 824 B1 qui consiste à éliminer une étape de granulation en présence d'alcool et à le remplacer par un liquide de mouillage composé de lactose ou de saccharose dans de l'eau purifiée comme indiquée ci-après.

Cette absence d'éthanol présente en particulier l'avantage de pouvoir effectuer une fabrication à grande échelle des systèmes bioadhésifs selon l'invention sans utiliser des équipements antidéflagrants qui sont obligatoires quand de l'alcool - est présent dans une étape quelconque d'un procédé industriel.

Les systèmes bioadhésifs selon l'invention sont à libération prolongée et peuvent en outre être enrobés par une couche soluble à libération immédiate et contenant le même principe actif; ceci est particulièrement intéressant pour les traitements analgésiques pour lesquels un effet double -immédiat et prolongé- est recherché.

La présente description porte également sur un procédé de préparation d'un système thérapeutique bioadhésif mucosal à libération contrôlée contenant au moins un principe actif, présentant un pourcentage de dissolution du principe actif supérieure à 70 % en 8 heures, comprenant au moins :
- une étape de mélange du principe actif avec des protéines naturelles, ces dernières représentant au moins 50 % en poids du principe actif, et avec des excipients et produits de charge comprenant au moins un polymère hydrophile et ;
- une étape de mélange avec un alkylsulfate de métal alcalin à concentration comprise entre 3,5 et 10 % en poids du système thérapeutique bioadhésif, de préférence entre 4 et 6 %.

Dans un mode de réalisation, le système thérapeutique bioadhésif est un comprimé bioadhésif mucosal à libération prolongée et dont la préparation comprend les étapes suivantes :
a) une étape de mélange du principe actif avec des protéines naturelles, ces dernières représentant au moins 50 % en poids du principe actif, et avec des excipients et produits de charge comprenant au moins un polymère hydrophile ;
b) une étape de mouillage du mélange obtenu en a) avec un liant de type sucre monohydrate ou polyol ;
c) une étape de séchage du mélange et de calibrage des grains obtenus ;
d) une étape de mélange des grains obtenus avec un alkylsulfate de métal alcalin à concentration comprise entre 3,5 et 10 % en poids du comprimé, de préférence entre 4 et 6 %.

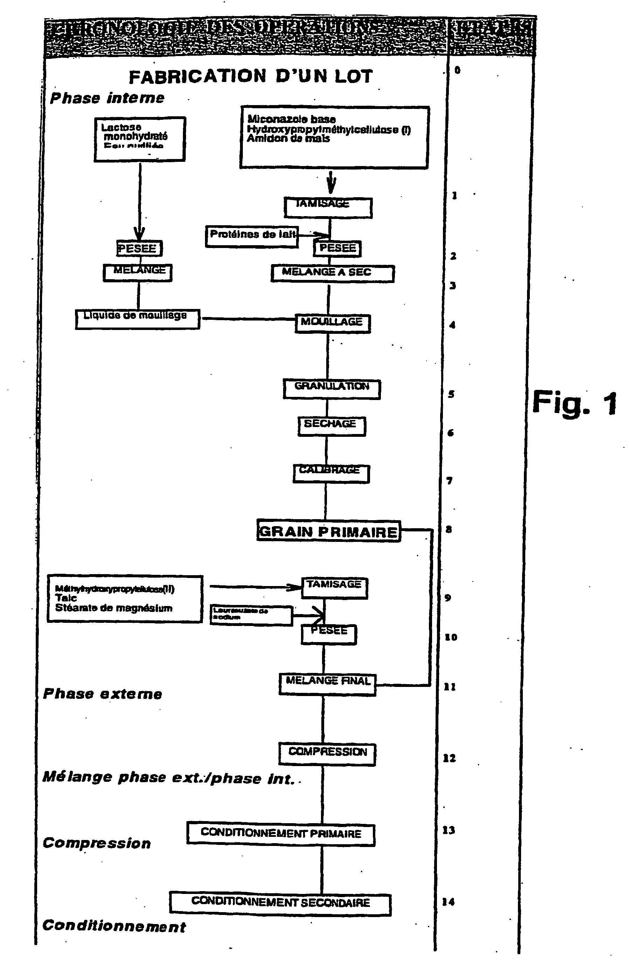

Dans le procédé, le ou les principes actifs, le cas échéant mélangés avec de méthylhydroxypropylcellulose ou métolose (MHPC), subissent un tamisage avec une ouverture de maille entre 0,4 et 1 mm, puis sont mélangés à au moins 50 % en poids de protéines naturelles, comme par exemple les protéines de lait. La poudre ainsi obtenue est homogénéisée. Elle subit ensuite une étape de mouillage en une étape de granulation dans laquelle la poudre est mélangée avec une solution de lactose ou de saccharose dans de l'eau distillée. Elle est ensuite suivie d'un séchage permettant d'atteindre une humilité résiduelle aux alentours de 3 %. Elle est ensuite suivie d'une étape de calibrage, de mélange aux excipients restants et d'une étape de compression. Une particularité du liquide de mouillage, lactose dans l'eau, est que la quantité introduite lors de la fabrication des lots n'est pas fixe mais est à déterminer en fonction de l'aspect du grain. Trois possibilités peuvent se présenter :
- soit tout le liquide de mouillage est introduit et les phases ultérieures de fabrication d'un comprimé sont réalisées classiquement par addition des excipients comme indiqué dans les exemples de réalisation ci-après ;
- soit tout le liquide de mouillage n'est pas mis en oeuvre et du métolose est ajouté avec les excipients de formulation pour arriver à une concentration finale (entre 0,1 et 1 % et de préférence entre 2 et 4 % du comprimé final). Un schéma type de fabrication est présenté dans la figure 1 illustrant l'exemple 2 ;
- soit la totalité du lactose ou du saccharose est dissoute dans la moitié de la quantité théorique d'eau purifiée. Tout ce liquide de mouillage est introduit dans le mélange. L'autre moitié d'eau purifiée restant est utilisée comme réserve lors de l'optimisation du mouillage et ajustable en fonction de la taille des lots.

Cette dernière possibilité permet d'éviter la phase de compensation du lactose monohydraté par du métolose. Elle est particulièrement avantageuse lorsqu'une transposition industrielle est recherchée car les quantités à introduire peuvent être calculées en connaissant la taille du lot sans se préoccuper de l'aspect du grain. Les expériences des transpositions industrielles ci-après indiquent que le procédé est approprié pour une fabrication à grande échelle et ce tant en présence de lactose que de saccharose. Les tests de dissolution à grande échelle avec la composition fournie dans l'exemple 3 en témoignent.

Une autre caractéristique essentielle du procédé de préparation des comprimés selon l'invention est l'addition d'un alkylsulfate de métal alcalin à l'étape d) ci-dessus à une concentration comprise entre 3,5 et 10 % en poids du comprimé et de préférence entre 4 et 6 %. Il est de fait ajouté en même temps que les excipients de formulation tel que le talc et le stéarate de magnésium, pour conférer aux comprimés les fonctions décrites ci-dessus, notamment sa vitesse de dissolution.

La description porte également sur un procédé de préparation d'un système thérapeutique bioadhésif autre que des comprimés, telles des microsphères, dans lequel une étape d'addition d'un alkylsulfate de métal alcalin est ajouté à la préparation dans les mêmes fourchettes de concentration que celles décrites ci-dessus.

L'alkylsulfate de métal alcalin est préférentiellement le laurilsulfate de sodium ou le diéthyl sulfosuccinate de sodium. La fabrication de lots pilotes décrits dans les exemples ci-après met en oeuvre le laurilsulfate de sodium à la concentration de 4,5 %.

Le procédé conduit à des comprimés bioadhésifs, à libération prolongée, particulièrement appropriés pour une application sur les muqueuses, et plus particulièrement à la muqueuse buccale.

Les exemples fournis portent sur des comprimés contenant à titre de principe actif des substances antifongiques pour le traitement des candidoses buccales, des substances anti-infectieuses (AZT, aciclovir), des principes actifs pour le traitement de la douleur (fentanyl) ou des nausées (métoclopramide).. L'homme du métier saura parfaitement adapter la fabrication du système thérapeutique bioadhésif avec un autre principe actif pour application à d'autres types de muqueuses et/ou à d'autres types de pathologies.

Pour les principes actifs présentant une absorption, deux mécanismes d'action, local et systémique, pourront être obtenus. On peut citer les aphtes pour les infections buccales, les anti-infectieux pour les infections vaginales, l'inflammation, les antalgiques locaux ou les analgésiques opioïdes pour la douleur, la sécheresse, le métoclopramide pour les nausées, les antiulcéreux et l'infection bactérienne locale.

La présente invention porte enfin sur l'utilisation des systèmes thérapeutiques bioadhésifs pour solubiliser les principes actifs et permettre une à libération prolongée comme médicament pour le traitement préventif, curatif ou d'entretien des pathologies, notamment des muqueuses. Plus particulièrement, l'intérêt des systèmes thérapeutiques bioadhésifs selon l'invention est leur utilisation pour prévenir ou traiter des infections à candidose de la muqueuse buccale et le système thérapeutique bioadhésif contient un azolé comme principe actif à la dose de 10 à 150 mg par comprimé et de préférence 25 à 75 mg par comprimé.

Pour le traitement des autres infections buccales, vaginales ou générales et plus particulièrement des infections à VIH (virus de l'immunodéficience humaine), à EBV (Virus Epstein-Barr, mononucléose infectieuse, leucoplasie chevelue), CMV (cytomégalovirus), de l'herpès (HSV), ou du virus de la varicelle et du zona (VZV), les principes actifs contenus dans les systèmes thérapeutiques bioadhésifs sont la zidovudine, l'aciclovir, le valaciclovir ou le ganciclovir. Les doses d'aciclovir et de valaciclovir sont de 20 à 100 mg, de préférence 50 mg. Les doses pour la zidovudine et le ganciclovir sont de 10 à 2000 mg, de préférence de 500 à 1500 mg. L'action recherchée privilégiée est locale et plus particulièrement au niveau de la voie d'entrée de l'agent infectieux mais aussi générale compte tenu de la particularité du mode d'action de ces virus à tropisme ganglionnaire. ,

L'utilisation des systèmes thérapeutiques bioadhésifs peut également porter sur un médicament pour le traitement des aphtes ou de la douleur. Pour le traitement de la douleur, l'action systémique est plus avantageusement recherchée associée à une libération locale et est obtenue avec le système thérapeutique bioadhésif grâce à son mode de libération. La faible biodisponibilité du fentanyl est améliorée par la forme bioadhésive solubilisant et libérant le principe actif au niveau de la muqueuse buccale. Ce système bioadhésif appliqué au fentanyl permet d'associer une action immédiate à une action prolongée particulièrement intéressante dans la douleur et en particulier les douleurs intenses, résistantes, notamment celles associées au cancer.

Le système bioadhésif selon l'invention est particulièrement intéressant quand le principe actif est le fentanyl base qui est une molécule insoluble et dont la solubilité est améliorée par le laurylsulfate. Une gamme de doses en fentanyl, de 50 à 1600 µg et de préférence 200 et 1200 µg, est possible avec ce système thérapeutique, permettant une adaptation à la douleur à traiter.

L'utilisation des systèmes thérapeutiques bioadhésifs selon l'invention est particulièrement attrayante pour le patient dans la mesure où la libération prolongée permet une administration unique du système thérapeutique bioadhésif une fois par 24 heures et que celui-ci permet une mobilisation locale du principe actif et un faible passage systémique. Les différents avantages du système thérapeutique bioadhésif et en particulier du comprimé selon l'invention préparé par le procédé décrit ci-avant apparaissent dans les exemples décrits ci-après illustrés des figures.

La figure 1 représente un schéma type de fabrication d'un comprimé selon l'invention.

La figure 2 représente un test de dissolution *in vitro* d'un comprimé à deux dosages différents (50 mg et 100 mg) contenant du laurilsulfate de sodium à la concentration de 4,5 %.

La figure 3 représente la concentration salivaire moyenne obtenue au cours du temps de miconazole en comparant un comprimé contenant 50 mg de miconazole (triangle noir), un comprimé contenant 100 mg de miconazole (carré blanc) et un gel contenant du miconazole et commercialisé sous la marque Daktarin ® (ronds blancs).

La figure 4 montre la corrélation entre le taux salivaire de miconazole exprimé en µg/ml et le pourcentage de dissolution *in vitro.*

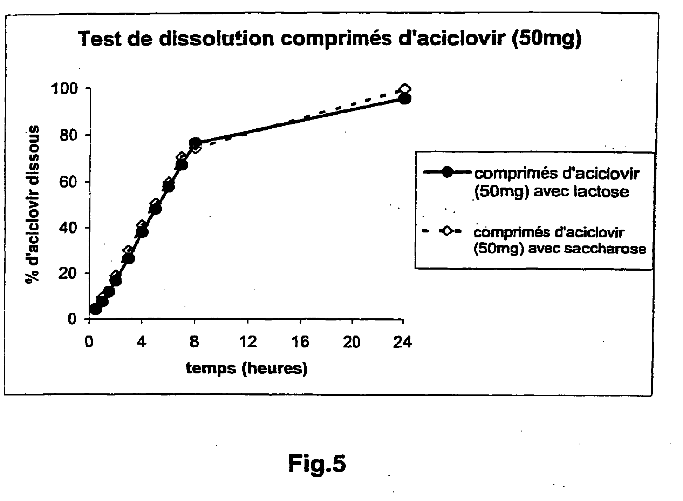

La figure 5 représente des test de dissolution *in vitro* de comprimés dosés à 50 mg d'aciclovir contenant du laurilsulfate de sodium à 4,5% et du lactose ou du saccharose comme liant.

### EXEMPLE 1 - EFFET DU LAURILSULFATE DE SODIUM SUR LE TEST DE DISSOLUTION DU COMPRIME BIOADHESIF :

Les caractéristiques de solubilité du miconazole dans une forme adhésive à libération prolongée ont été testées successivement par un test de dissolution dans les conditions de formulation classiques, telles que celles décrites dans EP 0 542 824, dans des conditions modifiées mais sans laurilsulfate puis avec addition de laurilsulfate de sodium au grain primaire dans les conditions de l'exemple 2 ci-après et de la figure 1.

### 1.1 Formule sans laurilsulfate de sodium :

Le test de dissolution effectué dans de l'eau pure donne, à des temps précoces (1 h et 3 h) des pourcentages de dissolution du miconazole très faibles (<5 %).

### 1.2 Formule sans laurilsulfate de sodium avec changement des conditions de réalisation du test :

Compte tenu de l'impossibilité de réaliser le test de dissolution dans l'eau pure et selon les recommandations de l'EMEA (Note for guidance on quality of modified release products), les conditions de réalisation du test de dissolution ont été modifiées afin d'obtenir le test *in vitro* le plus discriminant. Le test retenu satisfait toujours aux exigences de la Pharmacopée Européenne (Essai de dissolution des formes solides, Phée Eur 1997, 2.9.3) et se caractérise par un milieu de dissolution contenant 0,05 % de laurilsulfate de sodium.

### 1.3 Formule contenant du laurilsulfate de sodium :

Après ajout de 4,5 % de laurilsulfate de sodium dans le comprimé de miconazole, le test de dissolution donne des résultats satisfaisants pour une forme à libération prolongée.

Les résultats comparatifs des tests de dissolution dans les trois formules sont présentés dans le tableau I ci-dessous :

**TABLEAU I :**

| Temps (h) | Miconazole dissous (%) | | |
|---|---|---|---|
| | Formule 1.1 | Formule 1.2 | Formule 1.3 |
| 1 | < 5 % | 14,8 | 9,2 |
| 3 | < 5 % | 35,5 | 35,8 |
| 5 | ND | 52,2 | 61,1 |
| 8 | ND | 73,1 | 85,8 |

Dans la formule 1.2, le test de dissolution est amélioré par rapport à la formule 1.1 mais permet de conclure que la libération de principe actif est insuffisante pour un médicament à libération prolongée.

La formule 1.3 avec laurilsulfate de sodium permet non seulement une libération prolongée du miconazole mais obtient des qualités nouvelles (mouillage, gonflement et solubilisation du principe actif pour des concentrations locales constantes *in situ*).

### EXEMPLE 2 - PREPARATION D'UN COMPRIME CONTENANT 50 MG DE MICONAZOLE :

### a) Lot de taille 1,9 kg :

Le schéma de préparation de ce comprimé est présenté dans la figure 1. 50 mg de miconazole base (ou 100 mg pour un comprimé à 100 mg), sont mélangés à 9,2 mg d'amidon de maïs et à 20,5 mg de méthyl-hydroxy-propyl-cellulose.

Le mélange est ensuite homogénéisé par tamisage et 27,43 mg de protéines de lait LR85F sont ajoutés et mélangés au mélange initial. Ce dernier est ensuite mouillé par un mélange de lactose monohydraté (lactose 200 Mesh à raison de 0,39 mg dans de l'eau purifiée). Lors de la fabrication du lot, tout le liquide de mouillage a été introduit, soit 0,39 mg par comprimé de 115 mg, ce qui représente 0,34 % en poids de l'ensemble des constituants. L'étape de mouillage est suivie d'une étape de granulation, de séchage et de calibrage dans les conditions classiques, ce qui permet d'obtenir ce qui est indiqué dans la figure 1 comme grain primaire.

A ce grain primaire est ajouté le mélange des excipients : talc, stéarate de magnésium, méthyl-hydroxy-propyl-cellulose, auxquels sont ajoutés après tamisage le laurilsulfate de sodium à la concentration de 4,5 %.

Le mélange final subit ensuite les phases classiques de compression et de conditionnement du comprimé.

La composition d'un comprimé dosé à 50 mg et celle d'un comprimé dosé à 100 mg sont données dans le tableau ci-dessous.

**TABLEAU II - Composition 1 :**

| | **Lot dosé à 50 mg** **Composition en mg/comprimé** | **Lot dosé à 100 mg** **Composition en mg/comprimé** | **%** |
|---|---|---|---|
| Miconazole base | 50,00 | 100,00 | 43,50 |
| Méthylhydroxypropylcellulose Metolose 90SII15000 | 20,50 | 41,00 | 17,80 |
| Protéines de lait LR85F® | 27,43 | 54,86 | 23,80 |
| Amidon de maïs | 9,20 | 18,40 | 8,00 |
| Lactose monohydraté (Lactose 200 Mesh) | 0,39 | 0,78 | 0,34 |
| Laurilsulfate de sodium | 5,18 | 10,35 | 4,50 |
| Stéarate de magnésium | 1,32 | 2,65 | 1,15 |
| Talc | 0,98 | 1,96 | 0,85 |
| Eau purifiée | QS | QS | |
| **TOTAL** | 115,00 | 230,00 | 100 |

Dans cette composition, les protéines de lait peuvent être choisies parmi tous les concentrés cités ci-dessus pourvu qu'ils titrent au moins 85 % de protéines.

Par ailleurs, le saccharose peut être substitué au lactose dans les mêmes conditions que l'exemple 7 ci-après.

### b) Transposition industrielle : lot de 10 kg :

Une transposition industrielle a été réalisée en respectant la même composition que celle du tableau II ci-dessus, pour la production d'un lot de 10 kg.

A grande échelle, le procédé de la figure 1 peut être adapté et simplifié :
- en introduisant une quantité fixe de lactose, sans compensation par du métolose ;
- en réalisant un calcul préalable de la quantité d'eau à introduire, ce qui permet de ne pas se préoccuper de l'aspect du grain, rendant ainsi le procédé adaptable à des appareillages en cuves fermées.

Les tests de dissolution réalisés sur les compositions de l'exemple 3 ci-après indiquent la faisabilité industrielle du procédé à grande échelle, dans la mesure où ils ne diffèrent pas dans leurs résultats de ceux réalisés sur les comprimés issus du lot de 1,9 kg.

### EXEMPLE 3 - TEST DE DISSOLUTION IN VITRO :

### a) Comparaison des comprimés dosés à 50 et 100 mg/comprimé :

La libération du miconazole à partir de sa forme galénique ainsi que le taux de désagrégation du comprimé ont été mesurés *in vitro* par un test de dissolution. Le test est réalisé dans un appareil à palettes tournant à 60 trs/min, à 37° C dans un milieu adapté suivant les recommandations de la Pharmacopée Européenne. Le miconazole est dosé par la technique HPLC.

Les résultats obtenus pour les deux types de comprimés BA001-50 et BA001-100 sont rassemblés dans le tableau III ci-après. Seuls les pourcentages de miconazole libéré sont exprimés dans ce tableau ; en effet, les résultats des pourcentages de désagrégation du comprimé sont tout à fait superposables.

**TABLEAU III :**

| **Temps (h)** | **miconazole dissous (en %)** | |
|---|---|---|
| | **Comprimé à 50 mg** | **Comprimé à 100 mg** |
| 1 h | 9,2 ± 1,7 | 7,8 ± 0,5 |
| 3 h | 35,8 ± 4,6 | 30,2 ± 1,3 |
| 5 h | 61,1 ± 6,2 | 51,5 ± 2,0 |
| 8 h | 85,8 ± 3,5 | 74,4 ± 1,7 |

Ces études montrent qu'au bout de 8 heures, la quasi-totalité du miconazole est libérée de sa forme galénique pour le comprimé à 50 mg et la libération est constante. Les résultats obtenus avec le comprimé à 100 mg sont du même ordre. Ces temps de libération sont longs, laissant envisager *in vivo* une libération prolongée dans la cavité buccale procurant un effet antifongique prolongé. C'est cette hypothèse qui est vérifiée par l'étude clinique de phase I chez le volontaire sain dans l'exemple 4 ci-après. Cette étude est réalisée avec les deux dosages en miconazole (50 et 100 mg). Ainsi, en confrontant les temps de libération respectivement obtenus *in vitro* et les concentrations salivaires minimales souhaitées, un des deux dosages pourra être retenu et son rythme d'administration déduit.

### b) Comparaison d'un lot de taille laboratoire et d'un lot de taille industrielle.

Les quantités respectives de produits sont les suivantes :

| Comprimés à 50 mg de miconazole | Quantités (g) | |
|---|---|---|
| Matières premières | Lot de 1,9 kg (17.000 comprimés) | Lot de 9,8 kg (85.000 comprimés) |
| Miconazole base | 850,00 | 4261,00 |
| Métolose | 348,5 | 1747,00 |
| Concentré de protéines de lait | 466,31 | 2337,00 |
| Amidon de maïs | 156,4 | 784,00 |
| Lactose monohydrate | 6,63 | 34,00 |
| Lauryl sulfate de sodium | 88,06 | 441,00 |
| Stéarate de magnésium | 22,44 | 112,70 |
| Talc | 16,66 | 83,30 |
| Total | 1955,00 | 9800,00 |

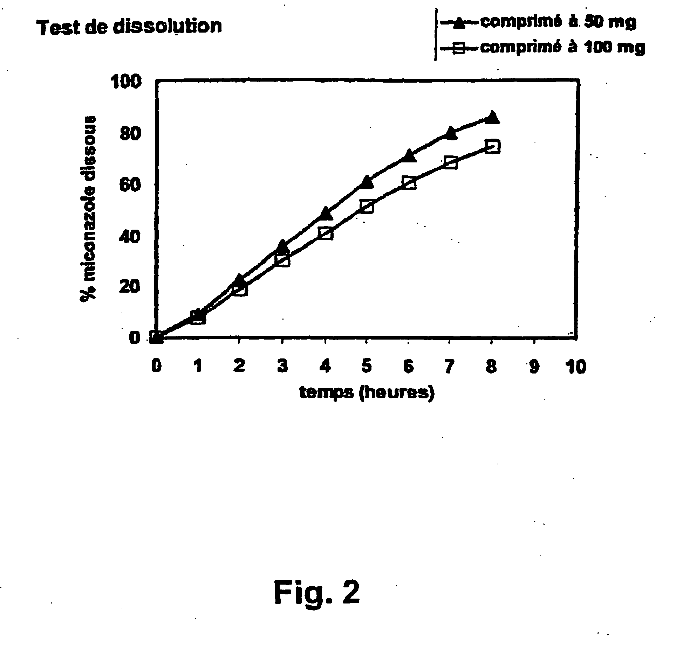

Les tests de dissolution obtenus pour les deux lots sont similaires à celui de la figure 2 avec plus de 70% de dissolution à 8 heures.

### EXEMPLE 4 - RESULTATS DES TESTS DE BIOADHESION AU TEXTUROMETRE :

Cet essai est destiné à comparer les propriétés adhésives des comprimés *in vitro.*

### Appareillage :

L'appareil utilisé pour cet essai est un texturomètre (de type TEC 025). Il se compose des éléments suivants :
- Un bras de levier qui permet les mouvements de montée et de descente de la sonde.
- Un capteur de force de 200 N qui mesure la force nécessaire à l'arrachement du comprimé
- Une sonde plastique à usage unique sur laquelle est fixée le comprimé
- Un module en acier surmonté d'un cristallisoir en plastique de 4 cm de diamètre, dont la base est constituées d'une plaque en acier inoxydable. Ce cristallisoir contiendra le milieu nécessaire au mouillage des comprimés.

### Mode opératoire:

- fixer le comprimé à étudier sur une sonde plastique avec de la colle cyanoacrylique
- visser la sonde sur le bras de levier du texturomètre
- placer de l'eau désionisée dans la cristallisoir (environ 4 ml)
- descendre la sonde dans le cristallisoir jusqu'à ce que le comprimé soit complètement immergé et laisser le comprimé dans l'eau pour permettre son mouillage pendant une durée définie
- procéder à la mesure de l'adhésivité in vitro en appliquant le protocole du texturomètre qui comprend 3 étapes :
   étape 1 : descente de la sonde jusqu'au contact du comprimé avec la plaque métallique ;
   étape 2: phase de contact qui permet l'adhésion du comprimé sur la plaque ;
   étape 3 : montée de la sonde : phase d'arrachement.

Dans l'exemple du tableau ci-dessous, les vitesses de montée et de descente de la sonde sont fixées à 0,25 mm/s, les temps de mouillage et de contact à 60 secondes, le milieu d'étude utilisé est de l'eau désionisée.

Les résultats sont les suivants :

| | Travail positif (J) | |
|---|---|---|
| Essais | Formule avec lactose | Formule avec saccharose |
| Comprimé 1 | 4,00.10⁻⁴ | 3,40.10⁻⁴ |
| Comprimé 2 | 3,78.10⁻⁴ | 3,18.10⁻⁴ |
| Comprimé 3 | 3,45.10⁻⁴ | 2,93.10⁻⁴ |
| Comprimé 4 | 4,06.10⁻⁴ | 3,25.10⁻⁴ |
| Moyenne | 3,82.10⁻⁴ | 3,19.10⁻⁴ |
| Ecart-type | 0,275.10⁻⁴ | 0,19.10⁻⁴ |

### Interprétation des résultats :

Les données mesurées par le capteur de force sont analysées par le logiciel Texturomètre TEC v6.0 ETIA 1996 et exprimées sous forme d'une courbe Force (N)/temps (s). Ce logiciel permet également de tracer une courbe Force/distance (mm). L'adhésivité in vitro des comprimés est évaluée par la valeur du travail positif (J) mesuré lors de la phase d'arrachement des comprimés. Ce travail représente l'aire sous la courbe Force/Distance.

Plus cette valeur est élevée, plus le comprimé a des propriétés bioadhésives importantes.

Les résultats obtenus dans des conditions identiques, avec des comprimés de même diamètre, vont permettre une comparaison entre différentes formulations.

### EXEMPLE 5 - ETUDE DE LA LIBERATION PROLONGEE IN VIVO AVEC LE COMPRIME DE L'INVENTION :

Cette étude est réalisée par une cinétique salivaire sur 18 volontaires sains et sur deux dosages en miconazole (50 et 100 mg). La tolérance de cette forme galénique est également évaluée.

Les comprimés selon l'invention sont comparés au gel buccal contenant du miconazole. Dans la mesure où ces gels ne résident pas dans la cavité buccale, ils doivent être appliqués plusieurs fois par jour. Les concentrations salivaires maximales de miconazole sont observées immédiatement après l'application, mais le principe actif est rapidement éliminé dans la cavité buccale (Odds, 1981, Clean Raise Rev. 1:231-232). Ainsi, après application de 6,25 g de gel buccal, équivalent à 125 mg de miconazole, les concentrations salivaires varient de 5 à 0,4 µg/ml de 30 min à 3 heures suivant une application. Le rythme d'administration est ainsi de quatre applications par jour.

### 5.1 Objectifs de l'étude :

### ● Objectif principal :

L'objectif principal de cette étude est de déterminer la cinétique salivaire du miconazole à partir de deux types de comprimés à libération prolongée (LP) à 50 ou 100 mg chez les dix-huit volontaires sains et de les comparer à celle obtenue avec le gel buccal (125 mg de miconazole à libération immédiate) dans les mêmes conditions.

### ● Objectif secondaire :

les objectifs secondaires de cette étude sont :
- de déterminer la pharmacocinétique sanguine du miconazole à partir de deux types de comprimés à libération prolongée (LP) à 50 ou 100 mg ;
- d'évaluer la tolérance clinique des comprimés bioadhésifs buccaux de miconazole ;
- d'apprécier l'acceptabilité et la tolérance du sujet lui-même.

### 5.2 Schéma de l'étude :

Etude monocentrique randomisée en cross-over de phase 1, chez le volontaire sain. Dix huit sujets recevront chacun les 3 types de produits dans un ordre différent avec une période de latence de 8 jours entre chaque administration.

Les 3 produits administrés sont le comprimé bioadhésif à 50 mg de miconazole, le comprimé bioadhésif à 100 mg de miconazole, et le gel à libération immédiate à 125 mg de miconazole par dose. Les traitements seront numérotés de 1 à 18. Dans chaque traitement, le produit est étiqueté en aveugle "semaine 1", "semaine 2" ou "semaine 3" (comprimé à 50 mg ou 100 mg ou gel).

Un dosage salivaire et sanguin du miconazole est effectué avant l'administration du produit. Ensuite un prélèvement salivaire et sanguin est effectué à 30 min. puis à 1 heure et ensuite toutes les heures jusqu'à 12 heures pour la salive ou jusqu'à 4 heures pour le sang avec ensuite un prélèvement sanguin à 8 et 12 heures ; pour la salive et pour le sang un prélèvement est effectué à 24 heures.

### ● Prèlevements :

- prélèvement salivaire: afin d'éviter des taux anormalement élevés de miconazole, la langue ne doit pas être au contact du comprimé pendant les 10 minutes précédant le prélèvement. De plus, pour les prélèvements ayant lieu 30 minutes après une administration, il faudra prendre soin de laver les lèvres du sujet;
   2 ml de salive sont collectés dans un tube borosilicaté sur une période de 2 min (1 min avant et 1 min après le temps donné) dans un tube borosilicaté.
   Les tubes étiquetés collectés sont conservés à -20° C en attendant d'être analysés.
- prélèvement sanguin : les tubes borosilicatés étiquetés collectés sont conservés à - 20° C en attendant d'être analysés.

### ● Modalités d'administration et adhésion des comprimés bioadhésifs :

- modalités d'administration : le comprimé prélevé dans le flacon unitaire sera prélevé et placé sous la lèvre supérieure dans les fosses canines. Pour ce placement, on utilisera soit les doigts, soit un dispositif à usage unique disponible permettant de centrer le comprimé dans la fosse canine et faciliter son adhésion.
- durée d'adhésion: la durée d'adhésion du comprimé est notée. Elle est définie comme le temps au bout duquel le comprimé n'est plus visible dans le vestibule ou sur la gencive avec un contrôle sensitif externe effectué au moment de chaque prélèvement. Les circonstances de la fin de l'adhésion doivent être spécifiées (érosion ou détachement du comprimé). En cas de détachement, le temps est noté et le comprimé doit être avalé.

### ● Modalités d'administration du gel buccal (selon la notice de Daktarin ® 2 % gel buccal) :

La posologie est de 2 cuillères-mesures fournie avec le tube de gel. Le gel doit être conservé dans la bouche le plus longtemps possible (2 à 3 minutes) avant de l'avaler. L'administration devra se faire à distance des repas ou de prise de boissons ou au moins 10 minutes après.

### ● Examen buccal :

Il est effectué au temps 10 h par la même personne que celle qui l'a réalisé avant l'administration du produit.

### 5.3 Evaluation :

### a) Critère d'évaluation principal :

### ● Pharmacocinétique salivaire de miconazole :

Le dosage de miconazole est effectué en aveugle par une technique de chromatographie liquide haute performance. Les résultats sont exprimés selon les critères habituels de pharmacocinétique (C_{max,} Tₘₐₓ, AUC) et les produits sont comparés. Les résultats apparaissent dans le tableau IV ci-après.

### b) Critères secondaires :

### ● Dosage sanguin du miconazole :

Le dosage de miconazole est effectué en aveugle par une technique de chromatographie liquide haute performance. Les résultats sont exprimés selon les critères habituels de pharmacocinétique (C_{max,} T_{max,} AUC) et les produits sont comparés.

### ● Tolérance clinique locale du comprimé bioadhésif :

Elle est évaluée par un examen clinique de la muqueuse buccale effectué par le même investigateur 10 heures après l'administration.

La tolérance est aussi appréciée, pour chaque produit et le jour de chaque administration, par le volontaire lui-même à l'aide d'un questionnaire (quatre choix possibles : bonne, acceptable, modeste ou désagréable ; si désagréable, précisez).

### ● Acceptabilité du comprimé bioadhésif :

Le volontaire l'apprécie à l'aide d'un questionnaire, pour chaque produit et le jour de chaque administration (quatre choix possibles : bonne, acceptable, modeste ou désagréable ; si désagréable, précision demandée).

### 5.4 Résultats :

### a) Concentration salivaire obtenue in vivo :

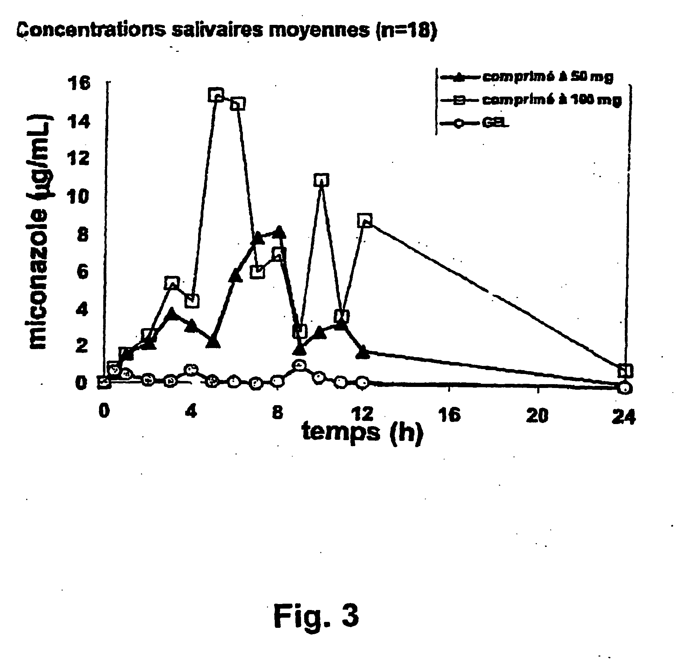

Les résultats des concentrations salivaires obtenues au cours du temps *in vivo* sont présentés dans la figure 3 et le tableau IV ci-dessous.

**TABLEAU IV :**

| Traitement | cₘₐₓ (µg/ml) | tₘₐₓ* (h) | AUC 0-12 (µgl.h/ml) |
|---|---|---|---|
| 100 mg de tablette Bioadhésive | 39,06±49,29 | 6 | 78,62±78,42 |
| 50 mg de tablette Bioadhésive | 15,07±16,21 | 7 | 42,97±31,99 |
| Gel | 1,61±1,62 | 4# | 3,43±4,14 |

| | | | |
|---|---|---|---|
| * = médiane ; # correspond à 0,5 heures après la deuxième administration. | | | |

Ces résultats indiquent que le comprimé dosé à 50 mg présente une concentration salivaire maximum de 15,07 µg/ml alors qu'elle est de 1,61 µg/ml pour le gel buccal. Les trois pics observés dans la figure 3 correspondent aux trois administrations du gel juste après T0, T4 et T9 alors que les comprimés ne sont administrés qu'une seule fois. Il apparaît donc que la concentration salivaire est très sensiblement supérieure avec les comprimés comparée à celle obtenue avec le gel buccal.

Les CMI (concentration minimale inhibitrice) du miconazole sur Candida albicans sont de 1 à 10 µg/ml. En observant les concentrations salivaires supérieures à 1 µg/ml, on constate que le gel dépasse rarement ce seuil alors que le comprimé bioadhésif à 50 mg est régulièrement supérieur à ce seuil (16 sujets sur 18 contre 0 avec le gel). Le temps moyen journalier pendant lequel le comprimé adhésif à 50 mg présente des taux salivaires supérieurs à 1 µg/ml est de 7,22 heures (versus 0,61 heures pour le gel). Cela signifie une concentration assurant localement une pression suffisante sur les éventuels germes et donc un risque d'apparition de souches résistantes moindre.

### b) Autres résultats :

Le temps moyen d'adhésion des comprimés est de 15 heures pour les comprimés à 50 et à 100 mg.

La concentration sanguine moyenne est de 0,035 µg/ml pour le gel, versus 0,020 µg/ml pour le comprimé à 50 mg et 0,013 µg/ml pour le comprimé à 100 mg.

La tolérance locale et générale est bonne, les comprimés adhésifs sont préférés dans 17 cas sur 18 (contre 1 sur 18 pour le gel).

Le goût s'est avéré un critère particulièrement noté dans un questionnaire spontané pour le gel (13 cas sur 18 signalent un mauvais goût, contre 2/18 pour le comprimé à 100 mg et 1/18 pour le comprimé à 50 mg).

### EXEMPLE 6 - CORRELATION IN VITRO / IN VlVO :

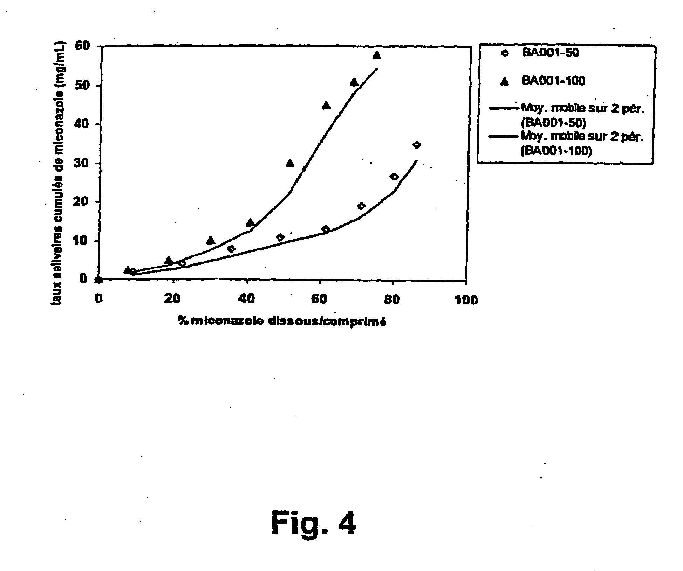

La figure 4 représente les taux salivaires cumulés de miconazole (µg/ml), en fonction du test de dissolution *in vitro* effectué sur 8 heures. Le test de dissolution *in vitro* est exprimé en abscisse sous la forme du rapport du pourcentage de miconazole dissous par rapport au pourcentage de miconazole dans le comprimé. Cette figure indique clairement que tout ce qui est libéré du comprimé se retrouve dans la salive. La déglutition est considérée comme constante et le passage systémique est nul. Dans la deuxième partie de la courbe dans laquelle la pente augmente, on dose plus de miconazole dans la salive que ce qui est libéré du comprimé.

Ce résultat est particulièrement important. En effet, l'hypothèse la plus vraisemblable qui puisse être faite au vu de ces résultats et compte tenu de l'absence de passage systémique, est celle d'un relargage de principe actif à partir de la muqueuse buccale. Ainsi, dans la première phase, il y aurait une déglutition avec perte de principe actif, associée à un stockage de principe actif dans la muqueuse buccale. Ce stockage s'explique par un équilibre du principe actif dans les micelles formées par le laurilsulfate, qui peut ensuite précipiter et se coller sur la muqueuse ; il est alors relargué au fur et à mesure que la salive est sécrétée.

Ainsi, les comprimés de la présente invention ont l'avantage non seulement qu'aucun passage systémique n'est observé mais qu'en plus la muqueuse buccale fait office de réservoir de principe actif. Ceci explique qu'une prise par 24 heures de 50 mg de principe actif est de façon surprenante largement suffisante alors que le gel buccal Daktarin ® est administré quotidiennement à 500 mg/j.

### EXEMPLE 7 : DIFFERENTES COMPOSITIONS REALI-SEES DES SYSTEMES BIOADHESIFS SELON L'INVENTION :

### 7.1 Modifications de principe actif :

La composition 1 du tableau II de l'exemple 2 a été reproduite avec d'autres principes actifs dans les conditions suivantes :

| Composition n° | Principe actif | Concentration mg/comprimé | Mode d'administration | Gamme de concentration autorisée |
|---|---|---|---|---|
| 1 | miconazole | 50 | Buccal ou vaginal | 2-100 |
| 2 | aciclovir | 50 | buccal ou vaginal | 2 - 100 |
| 3 | AZT | 700 | vaginal | 10 à 2000 préférence 500 à 1500 |
| 4 | métoclopramide | 100 | buccal | 20-100 |
| 5 | fentanyl | 1 | buccal | 0,05 -1,6 |
| 6 | Laurilsulfate | 5.18 | buccal ou vaginal | 1-100 |

### 7.2 Remplacement du lactose par le saccharose :

Dans toutes ces compositions, les 0,39 g par comprimé de lactose peuvent être substitués par 29,59 g ± 3 de saccharose.

Les résultats en tests de dissolution et tests d'adhésivité sont similaires.

### 7.3 Réalisation d'un comprimé à libération rapide et prolongée :

Les compositions ci-dessus peuvent être enrobées, quand la thérapeutique l'exige par une couche à libération rapide du même principe actif.

La géométrie de ces deux couches -libération rapide et libération prolongée- est indifférente et peut être réalisée par toutes les techniques connues du métier du moment que les qualités de bioadhésivité des comprimés de l'invention sont préservées.

### CONCLUSION :

La présente invention fournit ainsi un procédé de préparation et des comprimés bio-adhésifs à libération prolongée qui permettent l'administration d'un principe actif à raison d'une prise par 24 heures, à raison d'un dosage près de 10 fois inférieur à la forme galénique de référence proposée aujourd'hui. En outre, le test de dissolution, à savoir plus de 70 % en 8 heures est adapté au rythme d'administration. Dans le cas de miconazole, non seulement un faible passage systémique est observé mais, en outre, il semblerait que les concentrations *in situ* assurent une couverture antimycosique locale pendant un temps prolongé. La forme est adhésive pendant un temps prolongé et est bien tolérée, le goût et l'acceptabilité sont améliorés ce qui rend l'invention utile au plan clinique et extrapolable à de très nombreux principes actifs.

De façon plus générale, la présente invention est adaptable à d'autres systèmes mucoadhésifs de types microsphères ou nanosphères.

## Revendications

1. Système thérapeutique bioadhésif mucosal à libération prolongée pour utilisation comme médicament pour administration par voie mucosale, contenant au moins un principe actif, présentant un pourcentage de dissolution du principe actif supérieur à 70% en 8 heures, comprenant des quantités de protéines naturelles représentant au moins 50 % en poids de principe actif et au moins 20 % en poids dudit système thérapeutique bioadhésif, entre 10% et 20 % d'un polymère hydrophile, des excipients de compression, et comprenant entre 3,5% et 10 % d'un alkylsulfate d'un métal alcalin.

2. Système thérapeutique bioadhésif selon la revendication 1 **caractérisé en ce qu'**il s'agit d'un comprimé mucoadhésif.

3. Système thérapeutique bioadhésif selon la revendication 1 ou 2 **caractérisé en ce que** l'alkylsulfate de métal alcalin est le laurilsulfate de sodium ou le diethyl sulfosuccinate.

4. Système thérapeutique bioadhésif selon la revendication 3 dans lequel l'alkylsulfate est du laurilsulfate de sodium à la concentration de 3,5% à 10% et de préférence de 4% à 6 % en poids de l'ensemble des composés du comprimé.

5. Système thérapeutique bioadhésif selon la revendication 1, **caractérisée en ce qu'**il est sous forme de comprimé dont les excipients de compression contiennent de l'amidon de maïs.

6. Système thérapeutique bioadhésif selon l'une quelconque des revendications précédentes dans lequel un des principes actifs est un antifongique de la famille des azolés à large spectre, choisi de préférence parmi le miconazole, le clotrimazole, le kétoconazole, le fluconazole, l'itraconazole, l'isoconazole, l'econazole, le saperconazole, le genaconazole, le terconazole, le butoconazole, le tioconazole, l'oxiconazole, le bifonazole, le fenticonazole, l'omoconazole, le sertaconazole et le sulconazole.

7. Système thérapeutique bioadhésif selon la revendication 6 notamment un comprimé, dans lequel l'azolé est le miconazole présent à la dose de 10 à 150 mg par comprimé, de préférence 25 à 75 mg et encore de préférence à sensiblement 50 mg par système thérapeutique bioadhésif.

8. Système thérapeutique bioadhésif selon la revendication 6 dans lequel le principe actif est un azolé à large spectre, en association avec un autre principe actif choisi parmi :
- un antifongique à spectre différent, de type polyène,
- un analgésique,
- un agent de salivation,
- un antiseptique,
- un substitut salivaire,
- un anti-inflammatoire,
- un antibiotique,
- la thalidomide,
ou un mélange de ceux-ci.

9. Système thérapeutique bioadhésif selon la revendication 8 dans lequel le deuxième principe actif est un polyène à la dose de 10 à 100 mg par comprimé, de préférence de 20 à 90 mg.

10. Système thérapeutique bioadhésif selon la revendication 8 comprenant en outre 0,1% à 10 % en poids d'anesthésique et/ou 0,1% à 10 % en poids d'agent de salivation.

11. Système thérapeutique bioadhésif selon la revendication 1 ou selon la revendication 8 dans lequel le principe actif est un antiseptique.

12. Système thérapeutique bioadhésif selon la revendication 11 dans lequel l'antiseptique est le laurilsulfate de sodium à la concentration minimale de 3,5 % en poids.

13. Système thérapeutique bioadhésif selon la revendication 1 dans lequel le principe actif est un antiviral actif sur les virus herpès, virus varicelle-zona, virus Epstein-Barr, cytomegalovirus, choisi de préférence parmi l'aciclovir, le valaciclovir et le ganciclovir.

14. Système thérapeutique bioadhésif selon la revendication 13 notamment un comprimé, dans lequel le principe actif est présent soit de 20 à 100 mg, de préférence 50 mg pour l'aciclovir ou le valaciclovir, soit de 10 à 2000 mg, de préférence de 500 à 1500 mg pour la zidovudine et le ganciclovir.

15. Système thérapeutique bioadhésif selon la revendication 1 dans lequel le principe actif est un antiviral actif sur le virus de l'immunodéficience humaine, de préférence la zidovudine.

16. Système thérapeutique bioadhésif selon la revendication 15, notamment un système vaginal, dans lequel le principe actif est présent de 10 à 2000 mg par système thérapeutique bioadhésif, de préférence de 500 à 1500 mg.

17. Système thérapeutique bioadhésif selon la revendication 1 dans lequel le principe actif est un analgésique insoluble, de préférence le fentanyl.

18. Système thérapeutique bioadhésif selon la revendication 17, dans lequel le fentanyl, de préférence la base insoluble, est présent de 50 à 1600 microgrammes par système thérapeutique bioadhésif, de préférence 200 et 1200 microgrammes.

19. Système thérapeutique bioadhésif mucosal tel que défini à l'une quelconque des revendications 1 à 18 pour traiter ou prévenir une pathologie affectant une muqueuse, notamment les muqueuses buccales ou vaginales.

20. Système selon la revendication 19 dans lequel les pathologies des muqueuses sont des infections à candidose de la muqueuse buccale et le système thérapeutique bioadhésif contient un azolé comme principe actif.

21. Système selon la revendication 20 dans lequel l'azolé est le miconazole présent à la dose de 10 à 150 mg par système thérapeutique bioadhésif, de préférence 25 à 75 mg et encore de préférence à sensiblement 50 mg par système thérapeutique bioadhésif.

22. Système selon la revendication 19 pour le traitement local des aphtes.

23. Système selon la revendication 19 pour le traitement local de la douleur notamment des douleurs intenses associées au cancer, de l'inflammation ou des infections, notamment des mucites associées au cancer.

24. Système selon la revendication 19 pour le traitement des infections virales.

25. Système selon la revendication 24 dans lequel le principe actif est un anti-infectieux antiviral actif sur les virus herpès, virus varicelle-zona, virus Epstein-Barr, cytomegalovirus, choisi de préférence parmi l'aciclovir et le valaciclovir présent aux doses de 20 à 100 mg par comprimé, pour faciclovir et le métoclopramide, ou entre 10 à 2000 et de préférence 500 à 1500 mg pour l'AZT.

26. Système selon la revendication 24 dans lequel le principe actif est un anti-infectieux antiviral actif sur le virus de l'immunodéficience humaine, de préférence la zidovudine , dans lequel le principe actif est présent de 10 à 2000 mg par système thérapeutique bioadhésif, de préférence de 500 à 1500 mg.

27. Système selon la revendication 23 dans lequel le principe actif est le fentanyl et particulièrement le fentanyl base insoluble à une dose comprise entre 0,05 et 1,6 mg par comprimé pour le traitement des douleurs intenses et résistantes.

28. Utilisation d'un système thérapeutique contenant au moins un principe actif, et comprenant des quantités de protéines naturelles représentant au moins 50 % en poids de principe actif et au moins 20 % en poids dudit système thérapeutique bioadhésif, entre 10% et 20 % d'un polymère hydrophile, des excipients de compression, et comprenant entre 3,5% et 10 % d'un alkylsulfate d'un métal alcalin, pour la préparation d'un médicament bioadhésif mucosal à libération prolongée de principe actif présentant un pourcentage de dissolution du principe actif supérieur à 70% en 8 heures pour administration par voie mucosale.

29. Utilisation selon la revendication 28, **caractérisée en ce que** ledit système thérapeutique est tel que défini à l'une quelconque des revendications 2 à 27.

## Claims

1. A prolonged release bioadhesive mucosal therapeutic system for use as a drug for mucosal administration, containing at least one active principle, having an active principle dissolution test percentage of more than 70% over 8 hours, comprising quantities of natural proteins representing at least 50% by weight of active principle and at least 20% by weight of said bioadhesive therapeutic system, between 10% and 20% of a hydrophilic polymer, compression excipients, and comprising between 3.5% and 10% of an alkali metal alkylsulphate.

2. A bioadhesive therapeutic system according to claim 1, **characterized in that** it is a mucoadhesive tablet.

3. A bioadhesive therapeutic system according to claim 1 or 2, **characterized in that** the alkali metal alkylsulphate is sodium laurylsulphate or diethylsulphosuccinate.

4. A bioadhesive therapeutic system according to claim 3, in which the alkylsulphate is sodium laurylsulphate in a concentration of 3.5% to 10%, preferably 4% to 6% of the total weight of the compounds in the tablet.

5. A bioadhesive therapeutic system according to claim 1, **characterized in that** it is in the form of a tablet the compression excipients of which contain corn starch.

6. A bioadhesive therapeutic system according to any one of the preceding claims, in which one of the active principles is an antifungal from the broad spectrum azole family, preferably selected from miconazole, clotrimazole, ketoconazole, fluconazole, itraconazole, isoconazole, econazole, saperconazole, genaconazole, terconazole, butoconazole, tioconazole, oxiconazole, bifonazole, fenticonazole, omoconazole, sertaconazole and sulconazole.

7. A bioadhesive therapeutic system according to claim 6, in particular a tablet, in which the azole is miconazole present in a dose of 10 to 150 mg per tablet, preferably 25 to 75 mg and more preferably substantially 50 mg per bioadhesive therapeutic system.

8. A bioadhesive therapeutic system according to claim 6, in which the active principle is a broad spectrum azole, in association with a further active principle selected from:
• a polyene type antifungal with a different spectrum;
• an analgesic;
• a salivation agent;
• an antiseptic;
• a salivary substitute;
• an anti-inflammatory;
• an antibiotic;
• thalidomide;
or a mixture thereof.

9. A bioadhesive therapeutic system according to claim 8, in which the second active principle is a polyene in a dose of 10 to 100 mg per tablet, preferably 20 to 90 mg.

10. A bioadhesive therapeutic system according to claim 8, further comprising 0.1% to 10% by weight of anaesthetic and/or 0.1 % to 10% by weight of salivation agent.

11. A bioadhesive therapeutic agent according to claim 1 or claim 8, in which the active principle is an antiseptic.

12. A bioadhesive therapeutic system according to claim 11, in which the antiseptic is sodium laurylsulphate in a minimum concentration of 3.5% by weight.

13. A bioadhesive therapeutic system according to claim 1, in which the active principle is an antiviral that is active for herpes virus, varicella zona virus, Epstein-Barr virus or cytomegalovirus, preferably selected from aciclovir, valaciclovir and ganciclovir.

14. A bioadhesive therapeutic system according to claim 13, in particular a tablet, in which the active principle is present either in an amount of 20 to 100 mg, preferably 50 mg for acivlovir or valaciclovir, or 10 to 2000 mg, preferably 500 to 1500 mg for zidovudine and ganciclovir.

15. A bioadhesive therapeutic system according to claim 1, in which the active principle is an antiviral active against human immunodeficiency virus, preferably zidovudine.

16. A bioadhesive therapeutic system according to claim 15, in particular a vaginal system, in which the active principle is present in an amount of 10 to 2000 mg per bioadhesive therapeutic system, preferably 500 to 1500 mg.

17. A bioadhesive therapeutic system according to claim 1, in which the active principle is an insoluble analgesic, preferably fentanyl.

18. A bioadhesive therapeutic system according to claim 17, in which the fentanyl, preferably the insoluble base, is present in an amount of 50 to 1600 micrograms per bioadhesive therapeutic system, preferably 200 to 1200 micrograms.

19. A bioadhesive therapeutic system as defined in any one of claims 1 to 18, for the treatment or prevention of a disease affecting the mucosa, in particular buccal or vaginal mucosa.

20. A system according to claim 19, in which the mucosal diseases are candidiasis infections of the buccal mucosa and the bioadhesive therapeutic system contains an azole as the active principle.

21. A system according to claim 20, in which the azole is miconazole present in a dose of 10 to 150 mg per bioadhesive therapeutic system, preferably 25 to 75 mg and more preferably substantially 50 mg per bioadhesive therapeutic system.

22. A system according to claim 19, for the local treatment of aphthae.

23. A system according to claim 19, for the local treatment of pain, in particular intense pain associated with cancer, inflammation or infections, in particular mucitis associated with cancer.

24. A system according to claim 19, for the treatment of viral infections.

25. A system according to claim 24, in which the active principle is an antiviral anti-infectious agent that is active for herpes virus, varicella zona virus, Epstein-Barr virus or cytomegalovirus, preferably selected from aciclovir and valaciclovir, present in doses of 20 to 100 mg per tablet for acivlovir and metoclopramide, or 10 to 2000 mg, preferably 500 to 1500 mg, for AZT.

26. A system according to claim 24, in which the active principle is an antiviral anti-infectious agent active against human immunodeficiency virus, preferably zidovudine, in which the active principle is present in an amount of 10 to 2000 mg per bioadhesive therapeutic system, preferably 500 to 1500 mg.

27. A system according to claim 23, in which the active principle is fentanyl, in particular insoluble fentanyl base in a dose in the range 0.05 to 1.6 mg per tablet, for the treatment of intense resistant pain.

28. Use of a therapeutic system containing at least one active principle and comprising quantities of natural proteins representing at least 50% by weight of the active principle and at least 20% by weight of said bioadhesive therapeutic system, between 10% and 20% of a hydrophilic polymer, compression excipients, and comprising between 3.5% and 10% of an alkali metal sulphate, for the preparation of a prolonged release mucosal bioadhesive drug representing a percentage dissolution of active principle of more than 70% over 8 hours, for mucosal administration.

29. Use according to claim 28, **characterized in that** said therapeutic system is as defined in any one of claims 2 to 27.

## Patentansprüche

1. Bioadhäsives therapeutisches System für die Schleimhäute mit verzögerter Freisetzung zur Verwendung als Medikament zur Verabreichung auf die Schleimhäute, das mindestens einen Wirkstoff enthält, das einen Prozentsatz an Auflösung des Wirkstoffs in acht Stunden von über 70% zeigt, das Mengen an natürlichen Proteinen, welche mindestens 50% des Wirkstoffs und mindestens 20% des besagten bioadhäsiven therapeutischen Systems darstellen, zwischen 10% und 20% eines hydrophilen Polymers und Hilfsstoffe zum Verpressen umfasst, und das zwischen 3,5% und 10% eines Alkylsulfats eines Alkalimetalls umfasst.

2. Bioadhäsives therapeutisches System gemäß Anspruch 1, **dadurch gekennzeichnet, dass** es sich um eine mucoadhäsive Tablette handelt.

3. Bioadhäsives therapeutisches System gemäß Anspruch 1 oder 2, **dadurch gekennzeichnet, dass** das Alkylsulfat eines Alkalimetalls Natriumlaurylsulfat oder Diethylsulfosuccinat ist.

4. Bioadhäsives therapeutisches System gemäß Anspruch 3, in dem das Alkylsulfat Natriumlaurylsulfat ist in einer Konzentration von 3,5 % bis 10 % und vorzugsweise von 4 % bis 6 Gew.-% der gesamten Bestandteile der Tablette.

5. Bioadhäsives therapeutisches System gemäß Anspruch 1, **dadurch gekennzeichnet, dass** es in Form von Tabletten vorliegt, deren Hilfsstoffe zum Verpressen Maisamidon enthalten.

6. Bioadhäsives therapeutisches System gemäß einem der vorhergehenden Ansprüche, in dem einer der Wirkstoffe ein Breitband-Antipilzmittel der Azolfamilie ist, vorzugweise ausgewählt aus Miconazol, Clotrimazol, Ketoconazol, Fluconazol, Itraconazol, Isoconazol, Econazol, Saperconazol, Genaconazol, Terconazol, Butoconazol, Tioconazol, Oxiconazol, Bifonazol, Fenticonazol, Omoconazol, Sertaconazol und Sulconazol.

7. Bioadhäsives therapeutisches System gemäß Anspruch 6, insbesondere eine Tablette, wobei das Azol Miconazol ist, das in einer Dosis von 10 bis 150 mg pro Tablette, vorzugsweise von 25 bis 75 mg und noch weiter bevorzugt von ungefähr 50 mg pro bioadhäsivem therapeutischem System vorliegt.

8. Bioadhäsives therapeutisches System gemäß Anspruch 6, in dem der Wirkstoff ein Breitband-Azol in Verbindung mit einem anderen Wirkstoff ist, der ausgewählt ist aus:
- Antipilzmittel mit anderem Wirkspektrum vom Typ Polyen,
- Analgetikum,
- den Speichelfluss anregendes Mittel,
- Antiseptikum,
- Speichelsubstitut,
- entzündungshemmendes Mittel,
- Antibiotikum,
- Thalidomid,
oder ein Gemisch aus diesen.

9. Bioadhäsives therapeutisches System gemäß Anspruch 8, in dem der zweite Wirkstoff ein Polyen ist in einer Dosis von 10 bis 100 mg pro Tablette, vorzugsweise von 20 bis 90 mg.

10. Bioadhäsives therapeutisches System gemäß Anspruch 8, das außerdem 0,1 Gew.-% bis 10 Gew.-% eines Anästhetikums und/oder 0,1 Gew.-% bis 10 Gew.% eines den Speichelfluss anregenden Mittels umfasst.

11. Bioadhäsives therapeutisches System gemäß Anspruch 1 oder gemäß Anspruch 8, in dem der Wirkstoff ein Antiseptikum ist.

12. Bioadhäsives therapeutisches System gemäß Anspruch 11, in dem das Antiseptikum Natriumlaurylsulfat in einer Mindestkonzentration von 3,5 Gew.-% ist.

13. Bioadhäsives therapeutisches System gemäß Anspruch 1, in dem der Wirkstoff ein antivirales Mittel ist, das bei Herpesviren, Varicella Zoster-Viren, Epstein-Barr-Viren, Cytomegaloviren wirkt, vorzugsweise ausgewählt aus Aciclovir, Valaciclovir und Ganciclovir.

14. Bioadhäsives therapeutisches System gemäß Anspruch 13, insbesondere eine Tablette, wobei entweder 20 bis 100 mg, vorzugsweise 50 mg Wirkstoff für Aciclovir oder Valaciclovir, oder 10 bis 2000 mg, vorzugsweise 500 bis 1500 mg Wirkstoff für Zidovudin und Ganciclovir vorliegen.

15. Bioadhäsives therapeutisches System gemäß Anspruch 1, in dem der Wirkstoff ein antivirales Mittel ist, das beim Virus der humanen Immunschwäche wirkt, vorzugsweise Zidovudin.

16. Bioadhäsives therapeutisches System gemäß Anspruch 15, insbesondere ein vaginales System, in dem 10 bis 2000 mg Wirkstoff pro bioadhäsivem therapeutischem System vorliegen, vorzugsweise 500 bis 1500 mg.

17. Bioadhäsives therapeutisches System gemäß Anspruch 1, in dem der Wirkstoff ein unlösliches Analgetikum ist, vorzugsweise Fentanyl.

18. Bioadhäsives therapeutisches System gemäß Anspruch 17, in dem 50 bis 1600 Mikrogramm Fentanyl, vorzugsweise die unlösliche Base, pro bioadhäsivem therapeutischem System vorliegen, vorzugsweise 200 bis 1200 Mikrogramm.

19. Bioadhäsives therapeutisches System für die Schleimhäute, wie es in einem der Ansprüche 1 bis 18 definiert ist, zur Behandlung oder Vorbeugung einer Erkrankung, die die Schleimhäute betrifft, insbesondere die Mund- oder vaginalen Schleimhäute.

20. System gemäß Anspruch 19, in dem die Erkrankungen der Schleimhäute Candidose-Infektionen der Mundschleimhaut sind und das bioadhäsive therapeutische System ein Azol als Wirkstoff enthält.

21. System gemäß Anspruch 20, in dem das Azol Miconazol ist, das in einer Dosis von 10 bis 150 mg pro bioadhäsivem therapeutischem System vorliegt, vorzugsweise von 25 bis 75 mg und noch weiter bevorzugt von ungefähr 50 mg pro bioadhäsivem therapeutischem System.

22. System gemäß Anspruch 19 zur lokalen Behandlung von Aphthen.

23. System gemäß Anspruch 19 zur lokalen Schmerzbehandlung, insbesondere von intensiven Schmerzen, die mit Krebs, Entzündungen oder Infektionen in Verbindung stehen, insbesondere von Mucositis, die mit Krebs in Verbindung steht.

24. System gemäß Anspruch 19 zur Behandlung viraler Infektionen.

25. System gemäß Anspruch 24, in dem der Wirkstoff ein infektionshemmendes antivirales Mittel ist, das bei Herpesviren, Varicella Zoster-Viren, Epstein-Barr-Viren, Cytomegaloviren wirkt, vorzugsweise ausgewählt aus Aciclovir und Valaciclovir, die in Dosen von 20 bis 100 mg pro Tablette für Aciclovir und Metoclopramid, oder zwischen 10 und 2000 und vorzugsweise zwischen 500 und 1500 mg für AZT vorliegen.

26. System gemäß Anspruch 24, in dem der Wirkstoff ein infektionshemmendes antivirales Mittel ist, das beim Virus der humanen Immunschwäche wirkt, vorzugsweise Zidovudin, in dem 10 bis 2000 mg Wirkstoff pro bioadhäsivem therapeutischem System vorliegen, vorzugsweise 500 bis 1500 mg.

27. System gemäß Anspruch 23, in dem der Wirkstoff Fentanyl ist, und insbesondere die unlösliche Fentanyl-Base, in einer Dosis, die zwischen 0,05 und 1,6 mg pro Tablette liegt, zur Behandlung intensiver und hartnäckiger Schmerzen.

28. Verwendung eines therapeutischen Systems, das mindestens einen Wirkstoff enthält und das Mengen an natürlichen Proteinen, welche mindestens 50 Gew.-% des Wirkstoffs und mindestens 20 Gew.-% des besagten bioadhäsiven therapeutischen Systems darstellen, zwischen 10% und 20% eines hydrophilen Polymers und Hilfsstoffe zum Verpressen umfasst, und das zwischen 3,5% und 10% eines Alkylsulfats eines Alkalimetalls umfasst, zur Herstellung eines bioadhäsiven Medikaments für die Schleimhäute mit verzögerter Freisetzung des Wirkstoffs, das ein Prozentsatz an Auflösung des Wirkstoffs in acht Stunden von über 70% zeigt, zur Verabreichung auf die Schleimhäute.

29. Verwendung gemäß Anspruch 28, **dadurch gekennzeichnet, dass** das besagte therapeutische System derart ist, wie es in einem der Ansprüche 2-27 definiert ist.
